Europäisches Patentamt

European Patent Office

Office européen des brevets

(11) Numéro de publication: **0 385 874**
**A1**

## (12) DEMANDE DE BREVET EUROPEEN

(21) Numéro de dépôt: 90400579.0

(22) Date de dépôt: 02.03.90

(51) Int. Cl.5: **C07B 39/00, C07B 41/06, C07B 43/08, C07B 45/02, C07F 7/08, //C07C25/13, C07C43/225,C07C49/76, C07C255/54,C07C309/29**

(30) Priorité: 03.03.89 FR 8902756

(43) Date de publication de la demande:
05.09.90 Bulletin 90/36

(84) Etats contractants désignés:
AT BE CH DE DK ES FR GB GR IT LI LU NL SE

(71) Demandeur: **RHONE-POULENC CHIMIE**
**25, quai Paul Doumer**
**F-92408 Courbevoie Cédex(FR)**

(72) Inventeur: **Babin, Pierre**
**13, rue de Ségur**
**F-33000 Bordeaux(FR)**
Inventeur: **Benneteau, Bernard**
**3, rue Paul Verlaine**
**F-33740 Ares(FR)**
Inventeur: **Dunogues, Jacques**
**22, avenue du Président Pointcaré**
**F-33400 Talence(FR)**

(74) Mandataire: **Ricalens, François et al**
**RHONE-POULENC CHIMIE Service Brevets**
**Chimie 25, quai Paul Doumer**
**F-92408 Coubevoie(FR)**

(54) **Fonctionnalisation en position (n + 1) de dérivés aryliques disubstitués en position n et (n + 2).**

(57) La présente invention concerne un procédé de fonctionnalisation en position (n + 1) de dérivés aryliques disubstitués en position n et (n + 2) par des groupes ortho et paradirecteurs.
Le procédé consiste à former un intermédiaire aromatique organosilyle puis à fonctionnaliser ce composé.

EP 0 385 874 A1

## FONCTIONNALISATION EN POSITION (n + 1) DE DERIVES ARYLIOUES DISUBSTITUES EN POSITION n ET (n + 2)

La présente invention concerne un procédé de fonctionnalisation en position (n + 1) de dérivés aryliques disubstitués en position n et (n + 2). Elle concerne plus particulièrement la fonctionnalisation en position (n + 1) de dérivés aryliques substitués en position n et (n + 2) par des groupes ortho et para directeurs.

On entend par dérivés aryliques substitués en position n et (n + 2) par des groupes ortho et para directeurs les dérivés appartenant à la formule (I) suivante :

$$
\begin{array}{ccc}
R_1 & n + 1 & R_2 \\
n & & n + 2 \\
& & \\
R_3 & & R_5 \\
& R_4 &
\end{array}
\qquad (I)
$$

dans laquelle
- $R_1$ et $R_2$ sont des groupes identiques ou différents choisis parmi les radicaux : halogénoalcoxyle
amino- de préférence substitué
acylamino- de préférence substitué
- $R_3$, $R_4$, $R_5$ représentent des groupes identiques ou différents choisis parmi les radicaux hydrogéno-, alkyle, alkényle, alkylidène, alkénylidène, aryle, arylidène avec la condition que $R_3$, $R_4$, $R_5$ représentent au moins un hydrogène.

Il est connu de substituer des composés répondant à la formule (I) par substitution électrophile directe par exemple selon l'article de G. LOHAUS paru dans Organic Synthesis 1970, 50, 52.

Par exemple, lorsque l'on utilise comme matière première le diméthoxybenzène et qu'on effectue une cyanuration avec, comme agent cyanurant, l'isocyanate de chlorosulfonyle, on obtient exclusivement le cyano-6 diméthoxy-1, 3 benzène. La cyanuration a donc en lieu en ortho et para de chacun des groupes méthoxyle.

La substitution en ortho de chacun des deux groupes ortho et para directeurs portés par un même noyau benzénique n'avait donc pas lieu.

La présente invention a permis d'atteindre cet objectif.

Elle concerne un procédé de fonctionnalisation en position (n + 1) de dérivés aryliques disubstitués en position n et (n + 2) caractérisé en ce que :
- dans une première étape on effectue une anionisation en position (n + 1) dudit dérivé disubstitué,
- dans une deuxième étape sans traitement intermédiaire, on met en contact l'anion obtenu à la première étape avec un halogénure d'organosilyle,
- dans une troisième étape on met en contact le dérivé obtenu à la deuxième étape avec un réactif électrophile.

L'anionisation du dérivé disubstitué en position n et (n + 2) est réalisée, par exemple, par action d'un organolithien tel que le butyllithium, dans un solvant généralement aprotique polaire tel que le tétrahydrofuranne ou dans un alcane, généralement en présence d'un agent complexant comme, par exemple, la N,N,N′,N′-(tétraméthyl) éthylène diamine (TMEDA).

La deuxième étape du procédé de l'invention consiste à ajouter au milieu obtenu à la première étape un halogénure d'organosilyle.

On préfère, parmi les halogénures d'organosilyle, utiliser les halogénures de triméthylsilyle mais on peut utiliser, par exemple, des halogénures d'éthyldiméthylsilyle, de méthyldiéthylsilyle, de phényldiméthylsilyle etc. et, parmi les halogénures on préfère utiliser les chlorures.

La troisième étape du procédé selon l'invention, est effectuée par mise en contact du dérivé silicié obtenu à la deuxième étape avec un réactif électrophile. Cette étape permet une fonctionnalisation par échange entre le groupe silyle porté par le dérivé arylique et l'agent électrophile. Cet échange est effectué spécifiquement dans la position où se trouve le groupe silyle ce qui est couramment appelé l'effet "ipso"

EP 0 385 874 A1

du silicium. Cet effet est hélas erratique et n'est prévisible ni dans son existence ni dans son amplitude.

Lorsque les substituants du substrat de départ, c'est-à-dire faiblement ou moyennement donneurs par exemple halogène, l'échange entre le groupe silyle et l'agent électrophile peut se faire notamment avec l'ensemble des agents suivants :

- les acides carboxyliques ou sulfoniques
- les halogénures d'acides carboxyliques ou sulfoniques
- les anhydrides d'acides carboxyliques ou sulfoniques
- le brome, l'iode
- le chlorure d'iode
- le chlorosulfonate de triméthylsilyle
- les halogénures de tertiobutyle
- l'isocyanate de chlorosulfonyle

Lorsque les 2 substituants $R_1$ et $R_2$ sont fortement donneurs (alcoxy ou amino, de préférence linéaires) il convient d'enlever de cette liste les réactifs sulfoniques.

Le fonctionnalisation par les agents d'acylation est effectuée de préférence en présence d'un acide de Lewis.

L'acide de Lewis est choisi notamment parmi le chlorure d'aluminium, le trifluorure de bore, le tétrachlorure de titane et le tétrachlorure d'étain.

Les deux premières étapes du procédé selon l'invention sont mises en oeuvre de préférence dans un solvant aprotique polaire tel que le tétrahydrofuranne ou dans un solvant aliphatique saturé tel que les alcanes contenant 5 à 12 atomes de carbone.

La première étape de l'invention est réalisée de préférence à une température comprise entre - 80°C et 25°C.

La deuxième étape de l'invention est réalisée de préférence à une température comprise entre - 80°C et 50°C.

On utilise de préférence un rapport molaire du dérivé lithié au dérivé aromatique disubstitué d'environ 1 et un rapport molaire de la N,N,N',N'-(tétraméthyl) éthylène diamine au dérivé aromatique disubstitué d'environ 1.

Au cours de la deuxième étape on utilise une quantité d'halogénure d'organosilyle calculée en moles par rapport au dérivé aromatique disubstitué compris entre 0,8 et 1,5.

La troisième étape du procédé de l'invention est mise en oeuvre de préférence dans un solvant organique non basique. On peut citer à titre d'exemples, cette liste n'étant pas limitative :

- les alcanes,
- les halogénoalcanes,
- le disulfure de carbone,
- les nitroalcanes,
- les solvants haloaromatiques,
- les solvants nitroaromatiques.

Cette étape est réalisée de préférence à une température comprise entre - 50°C et 100°C. Les conditions opératoires seront adaptés à la nature des réactifs en présence et au solvant.

On utilise au cours de cette étape une quantité d'agent électrophile calculée en moles par rapport au dérivé aromatique disubstitué comprise entre 1 et 1,5.

Le produit obtenu à la fin de la troisième étape est extrait du milieu réactionnel par toute technique connue de l'homme de l'art notamment par extraction à l'aide de solvants.

Parmi les produits obtenus dans le cadre de la présente invention, on peut citer notamment :

- le diméthoxy - 2,6 cyanobenzène
- la difluoro - 2,6 acétophénone
- la dichloro - 2,6 acétophénone
- la diméthoxy - 2,6 acétophénone
- la chloro - 2, fluoro - 6, acétophénone
- le dichloro - 2,6 benzène sulfonate de sodium
- le difluoro - 2,6 benzène sulfonate de sodium
- le chloro - 2, fluoro - 6, benzène sulfonate de sodium
- le chloro -2, fluoro - 6, iodobenzène
- le dichloro - 2,6 iodobenzène
- le difluoro - 2,6 iodobenzène

La présente invention sera plus complètement décrite à l'aide des exemples suivants qui ne doivent en aucun cas être considérés comme limitatifs de l'invention.

3

Exemple 1 : FONCTIONNALISATION DU DIMETHOXY - 1,3 BENZENE

A - SILYLATION :

* Appareillage :

Un ballon de 100 ml à tubulure latérale équipé d'une agitation mécanique et d'une ampoule à brome isobare, balayé par un courant d'argon.

* Mode opératoire :

A une solution de 6,9 g (50 mmol) de diméthoxy-1,3 benzène dans 50 ml d'hexane, sont ajoutés goutte à goutte 6,4 g (50 mmol) de TMEDA à température ambiante. 22 ml d'une solution 2,5 M de n-BuLi dans l'hexane sont ensuite additionnés lentement à ce mélange à 0°C. L'agitation est poursuivie pendant 26 h à température ambiante. La formation d'un précipité jaune pâle est observée. Le mélange est de nouveau refroidi à 0°C puis 6 g (55 mmol) de Me₃SiCl sont ajoutés ; la température est maintenue à 0°C pendant 1 h. L'agitation est ensuite poursuivie à température ambiante pendant 18 h ; le précipité blanchit. Le mélange est versé dans 100 ml d'une solution saturée glacée de NH₄Cl. Après extraction à l'éther puis évaporation des solvants, on obtient, après recristallisation dans l'éthanol, 10 g de diméthoxy -2,6 triméthylsilylbenzène analysé par spectrographie infrarouge et résonnance magnétique nucléaire (F = 40°C).

B - ACYLATION

* Appareillage

Un ballon à tubulure latérale équipé d'une agitation mécanique et d'une ampoule à brome isobare, balayé par un courant d'argon.

* Mode opératoire

A 0,74 g (5,5 mmol) de chlorure d'aluminium (préalablement dégazé sous vide) en solution dans 10 ml de chlorure de méthylène sec à 0°C, sont ajoutés 0,43 g (5,5 mmol) de chlorure d'acétyle. L'agitation est poursuivie à 0°C pendant 15 mn. Le complexe est ensuite refroidi à -80°C. 0,94 g (4,5 mmol) de diméthoxy - 1,3 triméthylsilylbenzène en solution dans 5 ml de chlorure de méthylène sec est alors ajouté. Après agitation à -40°C pendant 2 h, le mélange est hydrolysé en le versant dans 50 ml d'une solution saturée glacée de NH₄Cl. La phase organique est séparée par décantation et la phase aqueuse lavée avec 30 ml de pentane. Les phases organiques sont rassemblées et lavées jusqu'à neutralité avec une solution de bicarbonate de sodium, puis séchées sur sulfate de sodium. Après évaporation des solvants, le produit acétylé est obtenu avec un rendement de 95 %.

La même opération est réalisée sur 4,5 mmol de diméthoxy-1,3 benzène en présence de butyllithium. On obtient le produit acétylé avec un rendement de 11 %.

C - CYANURATION

* Appareillage :

Un ballon à tubulaire latérale équipée d'une agitation mécanique et d'une ampoule à brome isobare, balayé par un courant d'argon.

* Mode opératoire :

1,37 g (9,7 mmol) d'isocyanate de chlorosulfonyle en solution dans 2 ml de chlorure de méthylène sont ajoutés goutte à goutte à 2,0 g (9,5 mmol) de diméthoxy-2,6 triméthylsilyl-1-benzène en solution dans 4 ml de chlorure de méthylène à 0°C.

L'addition terminée, l'agitation est poursuivie à température ambiante pendant 2 h. 1,43 g (19,5 mmol) de diméthylformamide (DMF) sont alors ajoutés et l'agitation maintenue pendant 1 h. Le milieu réactionnel est ensuite versé sur 10 g de glace ; lorsque celle-ci a fondu, 15 ml de chlorure de méthylène sont ajoutés. Après extraction et traitement habituel on obtient le produit attendu, après recristallisation dans l'éthanol (F = 120°C), avec un rendement non optimisé de 50 %.

D - SULFONATION (Exemple Comparatif)

* Appareillage :

Un ballon à tubulaire latérale équipé d'une agitation mécanique et d'une ampoule à brome isobare balayé par un courant d'argon.

* Mode opératoire :

Une solution de 1,7 g (9 mmol) de chlorosulfonate de triméthylsilyle dans 40 ml de $CCl_4$ est ajoutée goutte à goutte à une solution de 1,9 g (9 mmol) de diméthoxy - 2,6 triméthylsilylbenzène dans 40 ml de $CCl_4$ à température ambiante. Le mélange est chauffé au reflux pendant 48 h, puis hydrolysé avec 50 ml d'une solution de $NaHCO_3$. La phase organique est séparée par décantation et la phase aqueuse lavée avec 30 ml d'éther. Les phases organiques sont rassemblées et après évaporation des solvants, le produit est recristallisé dans l'éthanol après filtration à chaud avec un rendement de 62 % et identifié comme le diméthoxy 2,4 sulfonate de sodium.

E - IODURATION

* Appareillage :

Un ballon à tubulaire latérale équipé d'une agitation mécanique et d'un ampoule à brome isobare balayé par un courant d'argon.

* Mode opératoire :

1,5 g (9 mmol) de monochlorure d'iode en solution dans 10 ml de $CCl_4$ sont ajoutés goutte à goutte à une solution de 1,9 g (9 mmol) de diméthoxy -2,6 triméthylsilylbenzène dans 40 ml de $CCl_4$ à température ambiante. Le mélange est chauffé au reflux pendant 48 h. Après refroidissement il est versé dans 100 ml d'eau, puis extrait à l'éther. Les phases organiques sont rassemblées et lavées avec une solution aqueuse de $Na_2S_2O_3$ puis séchées. Après évaporation du solvant et recristallisation dans l'éthanol (F = 100°C), le produit iodé est obtenu avec un rendement de 95 %.

Exemple 2 : FONCTIONNALISATION DU CHLORO -1 FLUORO -3 BENZENE

A - SILYLATION DU CHLORO -1 FLUORO -3 BENZENE

* Appareillage :

on utilise le même qu'à l'exemple 1A

* Mode opératoire

A 3,3 g (25 mmol) de chloro-1 fluoro-3 benzène et 2,9 g (25 mmol) de TMEDA diluée dans 30 ml de THF, refroidie à -75° C, sont ajoutés lentement 10 ml d'une solution 2,5 M de n-BuLi dans l'hexane dilués dans 10 ml de THF.

L'agitation est poursuivie à cette température pendant 3 h. 2,7 g (25 mmol) de Me$_3$SiCl en solution dans 10 ml de THF sont ensuite additionnés, l'agitation est maintenue pendant 4 h à -75° C puis 15 h à température ambiante. Le mélange est ensuite versé dans 150 ml d'une solution saturée glacée de NH$_4$Cl. Après extraction à l'éther et évaporation des solvants, le produit silicié attendu est séparé par distillation (Eb$_{30\ mm}$ = 105° C)$_2$ avec un rendement de 80 %.

B - ACYLATION

* Appareillage :

on utilise le même qu'à l'exemple 1B.

* Mode opératoire :

On opère comme à l'exemple 1B avec le chloro-2 fluorotriméthylsilylbenzène. On obtient le produit acétylé avec un rendement de 55 %.

C - SULFONATION

On opére comme à l'exemple 1D. ON obtient alors le chloro-2 fluoro-6 benzène sulfonate de sodium avec un rendement de 13 %.

D - IODURATION

On opére comme à l'exemple 1E.
On obtient alors le chloro-2 fluoro-6 iodobenzène avec un rendement de 68 %.

Exemple 3 FONCTIONNALISATION DU DICHLORO-1,3 BENZENE

A - SILYLATION

En opérant comme à l'exemple 2A, on obtient, avec un rendement de 92 %, le dichloro 2,6 triméthylsilylbenzène (Eb 0,1 mm = 70° C)

B - ACYLATION

En opérant comme à l'exemple 2B, on obtient, avec un rendement de 94 % la dichloro 2,6 acetophénone.

En remplaçant les 5,5 moles de chlorure d'acétyle par 5,5 moles de chlorure de benzoyle, on obtient, avec un rendement de 83 %, la dichloro-2,6 benzophénone.

## C - SULFONATION

En opérant comme à l'exemple 2C, on obtient avec un rendement de 62 %, le dichloro-2,6 benzènesulfonate de sodium.

## D - IODURATION

En opérant comme à l'exemple 2D, on obtient, avec un rendement de 95 %, le dichloro-2,6 iodobenzène.

## Exemple 4 FONCTIONNALISATION DU DIFLUORO-1,3 BENZENE

### A - SILYLATION

En opérant comme à l'exemple 2A, on obtient, avec un rendement de 90 %, le difluoro 2,6 triméthylsilylbenzène (Eb$_{30 mm}$ = 70° C).

### B - ACYLATION

En opérant comme à l'exemple 2B on obtient, avec un rendement de 59 %, la difluoro-2,6 acétophenone.

### C - SULFONATION

En opérant comme à l'exemple 2C on obtient, avec un rendement de 11 %, le difluoro-2,6 benzènesulfonate.

### D - IODURATION

En opérant comme à l'exemple 2D on obtient, avec un rendement de 67 %, le difluoro-2,6 iodobenzène.

## Revendications

1. Procédé de substitution en position (n + 1) de dérivés aryliques disubstitués en position (n) et (n + 2) par des groupes o et p. directeurs caractérisé en ce que :
- dans une première étape on anionise en position (n + 1) ledit dérivé disubstitué,
- dans une deuxième étape sans traitement intermédiaire, on met en contact l'anion obtenu à la première étape avec un halogénure d'organosilyle,
- dans une troisième étape, on met en contact le dérivé obtenu à la deuxième étape avec un réactif électrophile.

2. Procédé selon la revendication 1 caractérisé en ce que l'anionisation est réalisée avec un mélange de butyllithium et de N,N,N′,N′-(tétraméthyl) éthylène diamine.

3. Procédé selon la revendication 1 caractérisé en ce que l'halogénure d'organosilyle est un halogénure de triméthylsilyle.

4. Procédé selon la revendication 1 caractérisé en ce que dans la troisième étape, le réactif électrophile est choisi parmi les acides, les halogénures d'acides carboxyliques ou sulfoniques, les anhydrides d'acides carboxyliques ou sulfoniques, le chlorosulfonate de triméthylsilyle, l'isocyanate de chlorosulfonyle, le brome, l'iode, le chlorure d'iode et les halogènes ou quelques dérivés halogénés comme des halogénures de tertiobutyle.

5. Procédé selon la revendication 4 caractérisé en ce que lorsque l'on met en oeuvre un acide, un halogénure ou un anhydride d'acide, on ajoute un acide de Lewis.

6. Procédé selon la revendication 1 caractérisé en ce que la condensation au cours de la 3e étape est effectuée à une température comprise entre -50 et 100°C.

7. Procédé selon la revendication 1 caractérisé au cours de la 3e étape on met en oeuvre un rapport molaire de l'agent électrophile au dérivé aromatique substitué compris entre 1 et 1,5.

Office européen
des brevets

**RAPPORT DE RECHERCHE EUROPEENNE**

Numero de la demande

EP 90 40 0579

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | THE JOURNAL OF ORGANIC CHEMISTRY, vol. 49, no. 24, 30 novembre 1984, pages 4657-4663, American Chemical Society, US; G.P. CROWTHER et al.: "Dilithiation of aromatic ethers" * Page 4662, colonne 1; page 4658, colonne 2, en bas; page 4659, colonne 1, en haut * --- | 1-7 | C 07 B 39/00<br>C 07 B 41/06<br>C 07 B 43/08<br>C 07 B 45/02<br>C 07 F 7/08 //<br>C 07 C 25/13<br>C 07 C 255/54<br>C 07 C 43/225<br>C 07 C 309/29<br>C 07 C 49/76 |
| Y | COMPTES RENDUS DE L'ACADEMIE DES SCIENCES, vol. 301, série II, no. 18, 7 décembre 1985, pages 1289-1292, Académie des Sciences; R. CALAS et al.: "Synthèse organique. - Sur la substitution électrophile régiosélective des arylsilanes et ses limitations" * En entier * --- | 1-7 | |
| Y | SYNTHESIS, no. 10, octobre 1988, pages 803-805; T.H. KRESS et al.: "Synthesis, stability, and reactions of 2,6-dichlorophenyllithium" * Page 804 * --- | 1-7 | **DOMAINES TECHNIQUES RECHERCHES (Int. Cl.5)**<br><br>C 07 B<br>C 07 F |
| A | CHEMICAL ABSTRACTS, vol. 74, no. 11, 15 mars 1971, page 314, résumé no. 53246u, Columbus, Ohio, US; G. LOHAUS: "2,4-Dimethoxybenzonitrile", & ORG. SYN. 1970, 50, 52-5 ----- | 1 | |

Le présent rapport a été établi pour toutes les revendications

| Lieu de la recherche | Date d'achèvement de la recherche | Examinateur |
|---|---|---|
| LA HAYE | 22-05-1990 | WRIGHT M.W. |